# EUROPÄISCHE PATENTANMELDUNG

(11) **EP 2 343 030 A1**
(43) Veröffentlichungstag der Anmeldung: **13.07.2011**
(21) Anmeldenummer: 11000041.1
(22) Anmeldetag: 05.01.2011
(51) Int. Cl.: A61F 2/44

(54) **Deckel für einen Wirbelkörperplatzhalter**

(30) Priorität: 11.01.2010 DE 102010004275
(71) Anmelder: Metz-Stavenhagen, Peter, 34537 Bad Wildungen (DE)
(72) Erfinder: Metz-Stavenhagen, Peter, 34537 Bad Wildungen (DE)
(74) Vertreter: Walther, Walther & Hinz GbR

(57) **Zusammenfassung**

Gegenstand der Erfindung ist ein Deckel für einen Wirbelkörperplatzhalter mit einem zylindrischen Innenkörper (12), der in einem koaxial angeordneten, hülsenförmigen Außenkörper (10) teleskopartig einschiebbar ist, wobei der Deckel (14) an einer Stirnseite des Innenkörpers (12) und/oder an einer Stirnseite des Außenkörpers (10) anbringbar ist, umfassend eine radial ausgerichtete Deckelwand (16) und ein in den Innenkörper (12) oder den Außenkörper (10) eingreifendes Führungselement (18). Einen Deckel für einen Wirbelkörperplatzhalter der eingangs genannten Art zu schaffen, der ein schnelles und einfaches platzieren, genauso wie ein schnelles und einfaches Ausrichten des Wirbelkörperplatzhalters ermöglicht wird dadurch erreicht, dass die Deckelwand (16) radial über das Führungselement (18) hinaus reicht, wobei das Mittel zum Befestigen des Deckels (14) an einem Wirbelkörper an dem über das Führungselement (18) hinausragenden Teil der Deckelwand (16) angebracht ist.

## Beschreibung

Die vorliegende Erfindung betrifft einen Deckel für einen Wirbelkörperplatzhalter gemäß dem Oberbegriff des Anspruches 1.

Aus der WO 2004/019827 A1 ist ein teleskopartig ausziehbarer Wirbelkörperplatzalter bekannt, dessen Stirnseite mit einem Deckel versehen werden kann. Dieser Wirbelkörperplatzhalter liegt nur stirnseitig, also von unten bzw. von oben, an den benachbarten Wirbelknochen an. In der Praxis kann es vorkommen, dass der Wirbelkörperplatzhalter nach einiger Zeit aus seiner angestammten Position herausrutscht. Dabei liegt der Erfindung die Erkenntnis zugrunde, dass ein Verrutschen des Wirbelkörperplatzhalters durch Befestigen des Wirbelsäulenplatzhalters an mindestens einem benachbarten Wirbelknochen verhindert werden kann.

Aus der EP 0 950 388 A2 ist ein Wirbelkörperplatzalter bekannt, der einen zylindrischen Innenkorpus und einen oberen und einen unteren, über entsprechende Gewinde gehaltene Außenzylinder aufweist. Am Außenumfang der Außenzylinder können bei Bedarf Halteelemente angeschraubt werden, die gewinkelt ausgeführt sind und deren einer Steg vom Wirbelkörperplatzhalter radial nach außen führt, während der andere Steg koaxial ausgerichtet ist und neben dem Wirbelkörper an diesem zur Anlage kommt. Der koaxiale Steg ist so breit ausgeführt, dass zwei Befestigungsschrauben nebeneinander angebracht werden können. Damit der koaxiale Steg besser an den Wirbelkörper angelegt werden kann ist dieser in Umfangsrichtung gekrümmt ausgeführt. Hierdurch kann der Wirbelkörperplatzhalter passgenau an den benachbarten Wirbelkörpern befestigt werden.

Es hat sich allerdings als unpraktisch erwiesen, dass die Halteelemente während der Implantation an die örtlichen Gegebenheiten angepasst und anschließend am Wirbelkörper angeschraubt werden müssen.

Aus der US 5,360,430 A ist ein Wirbelkörperplatzhalter bekannt, an dessen Stirnenden ein ausziehbarer Winkel vorgesehen ist. Mit diesem Winkel kann der Wirbelkörperplatzhalter am Wirbelknochen befestigt werden. Um den Abstand des koaxial angeordneten Steges des Winkels von der Längsachse des Wirbelkörperimplantates den individuellen Bedürfnissen des Patienten anzupassen, müssen allerdings zunächst die Befestigungsschrauben gelöst und später wieder angezogen werden. Dieser Vorgang ist sehr mühselig.

Davon ausgehend liegt der vorliegenden Erfindung die Aufgabe zu Grunde, einen Deckel für einen Wirbelkörperplatzhalter der eingangs genannten Art zu schaffen, der ein schnelles und einfaches platzieren, genauso wie ein schnelles und einfaches Ausrichten des Wirbelkörperplatzhalters ermöglicht.

Als technische Lösung dieser Aufgabe wird erfindungsgemäß ein Deckel für einen Wirbelkörperplatzhalter der eingangs genannten Art mit den Merkmalen des Anspruches 1 vorgeschlagen. Vorteilhafte Weiterbildungen dieses Deckels sind den Unteransprüchen zu entnehmen.

Ein nach dieser technischen Lehre ausgebildeter Deckel hat den Vorteil, dass durch die Integration der Befestigungsmittel direkt am Deckel, der behandelnde Arzt keine zusätzlichen Tätigkeiten zur Anbringung oder Einstellung der Befestigungsmittel durchführen muss, denn zusammen mit dem Einsetzen des Deckels, wie dies bereits aus der WO 2004/019827 bekannt ist, stehen auch gleich die Befestigungsmittel zur Verfügung. Somit kann sich der behandelnde Arzt sich voll und ganz auf seine chirurgische Tätigkeit konzentrieren.

Ein weiterer Vorteil besteht darin, dass der Deckel durch die rotationssymmetrische Ausbildung der Deckelwand und des Führungselementes in jeder beliebigen Position an dem Wirbelkörperplatzhalter angebracht werden kann, so dass die Befestigungsmittel in einfache Weise an einer geeigneten Stelle des Wirbelkörpers ausgerichtet werden können. Dies geschieht normalerweise quasi ohne Mehraufwand beim Einsetzen des Deckels in den Innenkörper, bzw. in den Außenkörper. Somit ist ein einfaches und schnelles Einsetzen und Ausrichten des Wirbelkörperplatzhalters möglich.

Es hat sich als vorteilhaft erwiesen, den Befestigungssteg schmal und länglich auszubilden, denn so kann der Befestigungssteg an einer beliebigen Stelle am Wirbelkörper angebracht werden, ohne vorher mühsam an die individuellen Gegebenheiten angepasst werden zu müssen. Vorteilhafterweise besitzt der Befestigungssteg hierzu ein Höhen-Breiten-Verhältnis von mindestens 2, vorzugsweise 5.

In einer vorteilhaften Ausführungsform ist das Befestigungsmittel an einem radial über das Führungselement hinausstehenden Teil der Deckelwand angebracht. Dies hat den Vorteil, dass hierdurch das Befestigungsmittel in einfacher Weise um den Wirbelkörper herumgeführt werden kann.

Auch hat es sich als vorteilhaft erwiesen, den Befestigungssteg einstückig am Deckel des Wirbelkörperplatzhalters anzubringen, weil hierdurch ein aufwendige Montage entfällt.

Die Anbringung am äußersten Rand der Deckelwand hat den Vorteil, dass der Befestigungssteg hierdurch bereits seitlich vom eigentlichen Wirbelkörper angeordnet ist und somit eine einfache Anbringung erfolgen kann.

Noch Vorteilhafter ist es allerdings, zwischen Deckelwand und Befestigungssteg einen sich radial erstreckenden Verbindungssteg vorzusehen, weil der Befestigungssteg hierdurch noch weiter herausgelangt.

Weil der Befestigungssteg so schmal ausgeführt ist, können keine zwei Schraubenaufnahmelöcher nebeneinander angeordnet werden. Die Anordnung der Schraubenaufnahmelöcher untereinander hat dabei noch den Vorteil, dass der Wirbelkörperplatzhalter hierdurch Verwindungssteif am Wirbelkörper befestigt wird.

Weitere Vorteile des erfindungsgemäßen Deckels für einen Wirbelkörperplatzhalter ergeben sich aus der beigefügten Zeichnung und den nachstehend beschriebenen Ausführungsformen. Ebenso können die vorstehend genannten und die noch weiter ausgeführten Merkmale erfindungsgemäß jeweils einzeln oder in beliebigen Kombinationen miteinander verwendet werden. Die erwähnten Ausführungsformen sind nicht als abschließende Aufzählung zu verstehen, sondern haben vielmehr beispielhaften Charakter. Es zeigen:
- Fig. 1: eine Explosionsdarstellung eines erfindungsgemäßen Wirbelkörperplatzhalters mit den benachbarten Wirbelkörpern;
- Fig. 2: eine geschnitten dargestellte Seitenansicht eines Deckels des Wirbelkörperplatzhalters gemäß Fig. 1, geschnitten entlang Linie II-II;
- Fig. 3: eine geschnitten dargestellte Seitenansicht einer zweiten Ausführungsform eines Deckels eines Wirbelkörperplatzhalters.

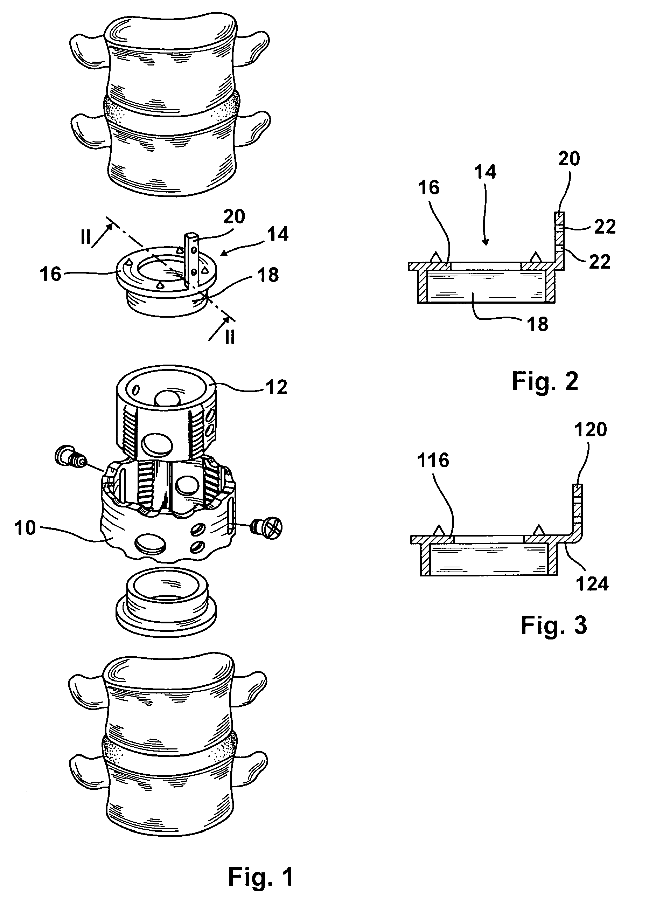

In den Fig. 1 und 2 ist eine erste Ausführungsform eines erfindungsgemäßen Wirbelkörperplatzhalters dargestellt, der einen hülsenförmigen Außenkörper 10 und einen axial verschieblich darin gehaltenen hülsenförmigen Innenkörper 12 umfasst, wobei die obere und die untere Stirnseite mit einem Deckel 14 versehen ist. Der Deckel 14 besitzt eine im Wesentlichen radial ausgerichtete Deckelwand 16, an der ein umlaufendes Führungselement 18 angeformt ist, welches in den Außen- 10 bzw. den Innenkörper 12 einführbar ist.

Am äußersten Rand der Deckelwand 16 ist einstückig ein axial ausgerichteter Befestigungssteg 20 angeformt, der zwei übereinander angeordnete Schraubenaufnahmelöcher 22 aufweist. Dabei ist der Befestigungssteg 20 derart schmal und länglich ausgeführt, dass gerade eben die Schraubenaufnahmelöcher 22 angebracht werden können.

In der in Fig. 3 dargestellten zweiten Ausführungsform ist zwischen dem Befestigungssteg 120 und der Deckelwand 116 ein radial ausgerichteter Verbindungssteg 124 vorgesehen, der ebenfalls einstückig angeformt ist.

Der Deckel 14 ist mit seinem Führungselement 18 drehbar im Innen- 12 oder im Außenkörper 10 gehalten. Außerdem kann der schlanke und längliche Befestigungssteg 20 an jeder beliebigen Stelle am Korpus angebracht werden. Dabei kommt dem Befestigungssteg 20 seine schlanke Gestalt mit einem Höhen-Breiten-Verhältnis von mindestens 2, vorzugsweise 5 zugute, denn ein derartig schlanker Befestigungssteg 20 kann ohne an die Kontur des Wirbelkörpers angepasst zu werden an jedem Punkt des Wirbelkörpers angeschraubt werden.

Während der Implantation des Wirbelkörpers setzt der behandelnde Arzt den Wirbelkörperplatzhalter zusammen mit dem daran angebrachten Deckel an den gewünschten Platz in der Wirbelsäule ein und dreht anschließend den Deckel 14 mit dem daran angebrachten Befestigungssteg 20 in die endgültige Position, bevor die Befestigungsschrauben in den Wirbelkörper eingesetzt werden.

### Bezugszeichenliste:

- 10: Außenkörper
- 12: Innenkörper
- 14: Deckel
- 16: 116 Deckelwand
- 18: Führungselement
- 20: 120 Befestigungssteg
- 22: Schraubenaufnahmelöcher
124 Verbindungssteg
126

## Patentansprüche

1. Deckel (14) für einen Wirbelkörperplatzhalter mit einem zylindrischen Innenkörper (12), der in einem koaxial angeordneten, hülsenförmigen Außenkörper (10) teleskopartig einschiebbar ist, wobei der Deckel (14) an einer Stirnseite des Innenkörpers (12) und/oder an einer Stirnseite des Außenkörpers (10) anbringbar ist, umfassend eine radial ausgerichtete Deckelwand (16, 116) und ein in den Innenkörper (12) oder den Außenkörper (10) eingreifendes Führungselement (18),
**dadurch gekennzeichnet,**
**dass** an der Deckelwand (16, 116) Mittel zum Befestigen des Deckels an einem Wirbelkörper vorgesehen sind.

2. Deckel nach Anspruch 1,
**dadurch gekennzeichnet,**
**dass** die Deckelwand (16, 116) radial über das Führungselement (18) hinaus reicht, wobei das Mittel zum Befestigen des Deckels (14) an einem Wirbelkörper an dem über das Führungselement (18) hinausragenden Teil der Deckelwand (16, 116) angebracht ist.

3. Deckel nach einem der vorangehenden Ansprüche,
**dadurch gekennzeichnet,**
**dass** das Mittel zum Befestigen des Deckels (14) an einem Wirbelkörper ein länglicher Befestigungssteg (20, 120) ist, der koaxial zu einer Längsachse des Wirbelkörperplatzhalters ausgerichtet ist.

4. Deckel nach einem der vorangehenden Ansprüche,
**dadurch gekennzeichnet,**
**dass** der Befestigungssteg (20, 120) einstückig an der Deckelwand (16, 116) angeformt ist.

5. Deckel nach einem der vorangehenden Ansprüche,
**dadurch gekennzeichnet,**
**dass** der Befestigungssteg (20) am äußeren Rand der Deckelwand (16) angebracht ist.

6. Deckel nach einem der vorangehenden Ansprüche,
**dadurch gekennzeichnet,**
**dass** der Befestigungssteg (20) ein Höhen-Breitenverhältnis von mindestens 2, vorzugsweise 5 aufweist.

7. Deckel nach einem der vorangehenden Ansprüche,
**dadurch gekennzeichnet,**
**dass** an der Deckelwand (116) ein sich radial ersteckender Verbindungssteg (124) angebracht ist, an dem der Befestigungssteg (120) gehalten ist.

8. Deckel nach einem der vorangehenden Ansprüche,
**dadurch gekennzeichnet,**
**dass** am Befestigungssteg (20, 120) zwei übereinander angeordnete Schraubenaufnahmelöcher (22) ausgebildet sind.

9. Deckel nach einem der vorangehenden Ansprüche,
**dadurch gekennzeichnet,**
**dass** an einer einem Wirbelknochen zugewandten Seite des Befestigungssteges ein Dorn vorgesehen ist.

10. Deckel nach einem der Ansprüche 6 bis 8,
**dadurch gekennzeichnet,**
**dass** der Verbindungssteg (124) rechtwinkelig zum Befestigungssteg (120) ausgerichtet ist.
